# EUROPÄISCHE PATENTSCHRIFT

(11) **EP 3 146 925 B1**
(45) Veröffentlichungstag und Bekanntmachung des Hinweises auf die Patenterteilung: **13.03.2019**
(21) Anmeldenummer: 16152496.2
(22) Anmeldetag: 22.01.2016
(51) Int. Cl.: A61B 18/02

(54) **KRYOSPITZE**
CRYOTIP
POINTE CRYOGENIQUE

(30) Priorität: 24.03.2015 DE 102015205367
(43) Veröffentlichungstag der Anmeldung: 29.03.2017
(73) Patentinhaber: PHARMPUR GmbH, 86343 Königsbrunn (DE)
(72) Erfinder: LINGENFELDER, Christian, 89081 Ulm (DE); LINDNER, Christopf, 89073 Ulm (DE)
(74) Vertreter: Leißler-Gerstl, Gabriele

(56) Entgegenhaltungen:
- EP-A1- 1 495 734
- US-A- 5 423 807
- US-A1- 2013 289 549

## Beschreibung

### Gebiet der Erfindung

Die Erfindung betrifft das Gebiet der Kryomedizin, insbesondere eine Kryospitze für das gezielte Setzen von Kälteherden zur kryotherapeutischen Behandlung von Gewebe.

### Hintergrund der Erfindung

Die Geschichte der Kryotherapie reicht zurück bis in das Jahr 2500 v. Chr. im Alten Ägypten, als Kälte verwendet wurde, um Verletzungen zu lindern. Die ersten dokumentierten Resultate einer kontrollierten Anwendung kältetherapeutischer Methoden, durch lokales Vereisen von Gewebe, stammen von J. Arnott, der mithilfe einer Kältemischung aus Eis und Kochsalz Temperaturen von bis zu -24°C zur Behandlung von Krebs erzeugte. In der Folge wurde innerhalb der medizinischen Forschung festgestellt, dass eine effektive Tumorbehandlung eine schnellere Vereisungsrate und somit tiefere Temperaturen des Kühlmittels erfordert. Die Arbeiten auf dem Gebiet der Gasverflüssigung von Linde und der Einsatz verflüssigter Luft von C. White zum medizinischen Gebrauch gegen Ende des 19. Jahrhunderts sind als Schlüsselereignisse für die breite Akzeptanz der Kryotherapie anzusehen. Weiterhin kam durch die Forschungsbeiträge von W. Pusey, Hall-Edwards und Cranston-Low kryogenes Kohlenstoffdioxid zum Einsatz, wodurch die Hauttemperatur bei der Behandlung auf -79°C erniedrigt werden konnte. Weitere Arbeiten zeigten, dass sich durch flüssigen Stickstoff, welcher im Vergleich zu verflüssigter Luft nicht explosiv ist, mit seinem niedrigen Siedepunkt Temperaturen von bis zu -196°C erreichen lassen. Die Entwicklung von Handgeräten für die Kryotherapie vereinfachte die medizinische Anwendung von Verfahren, in denen Kryosprays und Kryosonden verwendet werden. Heutzutage ist flüssiger Stickstoff das populärste kryogene Fluid. Weiterhin ist Kohlenstoffdioxid (Siedepunkt bei -79°C) aufgrund seiner einfachen Lagerung gefragt. Andere Kühlmittel sind Distickstoffmonoxid (Siedepunkt bei -89 C) und Halogenkohlenwasserstoffe mit einem Siedebereich zwischen -30°C und -41°C). Die Wahl des jeweiligen Kühlmittels hängt von der Art des zu behandelnden Gewebes ab (F.W. Frauenfelder, Trans. Am. Opthtalmol. Soc. / Vol 106 / 2008).

Die Kryotherapie dient hauptsächlich zur gezielten Gewebedestruktion oder -vereisung mit tiefen Temperaturen. Gebiete, auf denen kryotherapeutische Gerätschaften zur Behandlung von Gewebe eingesetzt werden können, sind zum Beispiel die Behandlung von Hautkrankheiten, Tumoren, unerwünschten Läsionen, kardialer Arrhythmie, Geschwüren, Ischämie, Parkinson. Weiterhin ist das Gebiet der Augenheilkunde zu nennen. Hierbei ist es sehr wichtig, dass lediglich das zu behandelnde Gewebe selektiv eingefroren und das umliegende gesunde Gewebe durch die Kryotherapie, so wenig wie möglich mechanisch oder thermisch beschädigt wird. Weiterhin ist es wichtig, den Ressourcenverbrauch zur Kälteerzeugung so gering wie möglich zu halten.

Die Methoden der Tieftemperaturphysik, welche für die Erzeugung der Temperaturen in der Kryomedizin notwendig sind, umfassen hauptsächlich die Gaskühlung durch den Joule-Thomson-Effekt, die Verdampfungskühlung und die Erzeugung von Kälte durch den Peltier-Effekt. Diese Methoden sind dem Fachmann wohlbekannt.

Bei der Applikation des kryogenen Mediums wird zwischen der thermoelektrischen, der direkten und der indirekten Variante unterschieden. Eine thermoelektrische Variante ist die Erzeugung von Kälteherden im Gewebe beispielsweise durch ein speziell konstruiertes Peltier-Element, welches sich den Peltier-Effekt zunutze macht und dazu verwendet wird, um die Linse während einer Kataraktoperation zu entfernen (M.L. Kwito, C.E. Kelman, The History of Modern Cataract Surgery). Derartige Einrichtungen eignen sich zu einer gezielten Kälteherderzeugung an Stellen, welche einfach zugänglich sind; jedoch ist deren Wirkungsgrad sehr gering, was unter anderem den Verbrauch an Kältemedium unnötig erhöht. Weitere Nachteile stellen der komplexe Aufbau und die Notwendigkeit teurer Materialien dar.

Die indirekte Variante der Kälteapplikation findet über Kryosonden statt, zum Beispiel in Form eines Katheters, welcher die tiefen Temperaturen über den Joule-Thomson-Effekt erreicht, wie beispielsweise in der Patentanmeldung WO 03/039388 offenbart. Da hier jedoch das Gas innerhalb des Katheters expandiert, bildet sich ein Temperaturgradient aufgrund des Wärmedurchgangs durch die Katheterwand aus, wodurch die Abkühlgeschwindigkeit niedriger ist. Dementsprechend eignen sich derartige Einrichtungen zur Vereisung von Gewebe, das nicht zu schnell abgekühlt werden darf, um umliegendes gesundes Gewebe beispielsweise vor thermischen Spannungen zu schützen. Außerdem weisen derartige Kryosonden einen komplexen Aufbau, eine aufwändige Peripherie und teure Ausgangsmaterialien auf, wodurch hohe Fertigungskosten resultieren. Der komplexe Aufbau stellt weiterhin eine Schwierigkeit für den Operateur bei der selektiven Setzung der Kältestellen dar.

Einige dieser Nachteile können durch die direkte Variante der Kryoapplikation, d. h. den direkten Kontakt zwischen dem kryogenen Medium und dem zu behandelnden Gewebe, überwunden werden. Als Lösung offenbart die Patentanmeldung WO 01/41683 einen Applikator in Form einer Kapillare, welche in die Gewebeschicht eingestochen werden kann und dort über Verdampfungskühlung eines Flüssiggases, wie etwa Distickstoffmonoxid, das zu behandelnde Gewebe selektiv vereist. Durch ein derartiges Verfahren ergeben sich allerdings zwei Nachteile: Zum einen muss die Kapillare tiefer in das Gewebe eingestochen werden, als es die Lage der zu behandelnden Stelle verlangt, da sich der Auslass für das kryogene Flüssiggas oberhalb der Einstichspitze befindet. Auf der anderen Seite expandiert das Flüssiggas bei der Verdampfungskühlung innerhalb des tieferliegenden Gewebes. Als Ableitungskanal dient lediglich die Außenseite der Applikationskapillare. Dem Fachmann ist jedoch bekannt, dass Stoffe bei der Verdampfung das Siebenhundertfache ihres ursprünglichen Volumens einnehmen können. Hierdurch besteht insbesondere bei empfindlichem Gewebe das Risiko des Auftretens eines Baro- oder Volutrauma, was irreversible Schäden an gesundem Gewebe durch die Raumforderung des Gases verursachen kann. Ein weiterer Nachteil ist die unerwünschte Kühlung höherliegender gesunder Gewebeschichten, wenn das Gas beim Auslass an der Außenseite der Kapillare in Kontakt mit diesen steht.

Die direkte Variante eignet sich vor allem bei zu behandelndem Gewebe, bei dem eine schnelle Abkühlung notwendig ist, um eine effektive Behandlung zu gewährleisten. Allerdings muss betont werden, dass das Risiko thermischer Spannungen, insbesondere bei empfindlichem Gewebe, zwischen den umliegenden gesunden Gewebeschichten und dem zu behandelnden Gewebe durch die rasche Abkühlung größer ist als bei der indirekten Variante.

Als weitere Möglichkeit zur direkten Gewebekühlung steht beispielsweise die Kryospraytherapie mit Flüssigstickstoff zur Verfügung (F.W. Frauenfelder, Trans. Am. Opthtalmol. Soc., Vol 106 (2008)). Dort wird z. B. die Kryospraytherapie zur oberflächlichen Behandlung von Augengewebe verwendet. Diese Therapie eignet sich aufgrund der angesprochenen Nachteile jedoch nur für eine Oberflächenbehandlung. Weiterhin ist die selektive Setzung einer Kältestelle, ohne gesundes umliegendes Gewebe zu beschädigen, nur bedingt möglich.

Die Erfordernisse an Operationsgerätschaften für minimalinvasive Eingriffe sind groß. Die Operationsgerätschaft muss möglichst klein konstruiert sein. Insbesondere bei kryotherapeutischen Anwendungen ergibt sich der zusätzliche Bedarf an Materialien mit guten Wärmeisolationseigenschaften, um das gesunde umliegende Gewebe zu schonen. Kleine Operationsgeräte für kryotherapeutische Anwendungen können derzeit lediglich aus Glas und Glaskeramik mittels Ultraschallkanalfräsung gefertigt werden, wobei es sich um Materialien mit schlechten Wärmeisolationseigenschaften handelt, was wiederum nachteilig für die Schonung von umliegendem und gesundem Gewebe ist.

Aus EP1495734 war ein Kryoapplikator mit mindestens einem Schaumstoffteil aus Kunststoff bekannt, das zur einer bestimmten Behandlungsgestalt zusammengepresst und gleichzeitig gekühlt werden kann.

Aufgabe der Erfindung ist es daher, eine möglichst einfach handhabbare und kostengünstige Einrichtung zur effektiven kryotherapeutischen Behandlung von Gewebe bereitzustellen, wobei selektiv Kältestellen oberflächlich oder in tiefer liegenden Gewebeschichten gesetzt werden können. Dabei soll eine hohe Flexibilität bei der Einstellung der Abkühlgeschwindigkeit für die entsprechende kryotherapeutische Anwendung möglich sein. Bei der Behandlung gilt es, das Risiko der mechanischen oder thermischen Beschädigung von umliegendem gesundem Gewebe zu minimieren. Eine weitere Aufgabe ist es, eine Spitze bereitzustellen, die aufgrund ihrer Abmessungen möglichst schonend bei der Anwendung, d. h. dass so fein wie möglich ist.

Diese Aufgaben werden mit einer Kryospitze gelöst, wie sie in Anspruch 1 definiert ist.

Folgende Definitionen werden für die Beschreibung und die Ansprüche der vorliegenden Erfindung verwendet:
Der Begriff "elastischer Rand" bezeichnet einen Rand aus elastischem Material, wie zum Beispiel einen O-Ring, welcher eine gasdichte Verbindung zwischen dem Abschluss der Kryospitze am distalen Ende und dem Gewebe schafft. Der elastische Rand kann Teil des distalen Endes der Kryospitze sein oder kann ein aufgesetzter Ring sein.

Der Begriff "distales Ende" bezeichnet den Endteil der Kryospitze, welches vom Operateur aus gesehen das entfernte Ende am Gewebe darstellt. Das distale Ende hat eine relative Länge zwischen 1/16 - 1/4 bezogen auf die Gesamtlänge der Kryospitze.

Die Bezeichnung "proximales Ende" bezieht sich auf das Endteil der Kryospitze, welches vom Operateur aus gesehen das sich nicht am Gewebe befindliche Ende darstellt. Das proximale Ende hat eine relative Länge von 1/16 - 1/2 bezogen auf die Gesamtlänge der Kryospitze.

"Kraftschlüssig" im Sinne der vorliegenden Erfindung ist eine Verbindung zwischen dem elastischen Rand des distalen Endes und dem zu behandelnden Gewebe durch Aufsetzen der Kryospitze. Der Kraftschluss zwischen Kryospitze und Gewebe wird lediglich durch die Normalkraft, welche auf der Kryospitze lastet und durch den Operateur aufbringbar ist, ermöglicht. Diese Verbindung verhindert einen Wärme- und Stoffaustausch mit dem umliegenden gesunden Gewebe; nur der Wärmeaustausch zwischen dem kryogenen Fluid und dem zu behandelnden Gewebe innerhalb des vom elastischen Rand eingeschlossenen Gebiets ist möglich.

Unter einem "kryogenen Fluid" werden ein tieftemperiertes Gas oder eine tieftemperierte Flüssigkeit verstanden, welche durch den direkten Kontakt mit dem zu behandelnden Gewebe Kältestellen auf dem Gewebe von -10°C bis zu -196°C erzeugen können.

Der Begriff "röhrenförmig" beschreibt einen länglichen Körper mit mindestens einer inneren Bohrung, welcher einen Querschnitt in beliebiger Form, z. B. eckig, ellipsenförmig, polygon, aufweist.

Mit "unter Druck stehend" wird der Zustand eines Kryofluids bezeichnet, das ausgehend von einem Druckbehälter über ein Entspannungsventil in die erfindungsgemäße Kryospitze expandiert wird. Der Druck des entspannten Fluids ist definitionsgemäß niedriger als der Druck des Fluids innerhalb des Druckbehälters. Wird beispielsweise N₂O als Kryofluid verwendet, das innerhalb des Druckbehälters unter 50 bar gelagert wird, so ist der Druck des entspannten Kryofluids innerhalb der Kryospitze entsprechend niedriger als 50 bar, wobei entsprechendes auch für andere Kryofluide gilt.

Die erfindungsgemäße modulare Kryospitze ermöglicht durch die Konstruktionsweise, dass zwischen einer direkten und einer indirekten Variante der Kryoapplikation gewählt werden kann. Bei der direkten Variante der Vereisung kann kein kryogenes Fluid an der kraftschlüssigen Verbindung zwischen der Kryospitze und zu dem behandelnden Gewebe entweichen, womit lediglich die zu behandelnde Stelle vereist wird und mit der restlichen Umgebung kein Stoff- und Wärmeaustausch stattfinden kann.

Bei empfindlichem Gewebe, das langsamer abgekühlt werden muss, kann die Öffnung der modularen Kryospitze entsprechend mit einer Metall- oder Diamantkappe verschlossen werden, um eine indirekte Variante der Kryotherapie zu ermöglichen. Die Metallkappe kann sowohl aus einem Metall als auch aus einer Legierung bestehen, kann mehrere Schichten aufweisen und bzw. oder mit einer Polymerschicht versehen sein. Der Operateur kann entsprechend der Art des zu behandelnden Gewebes oder der konkreten medizinischen Aufgabenstellung entscheiden, ob es sinnvoller ist die direkte oder die indirekte Vereisungsvariante zu wählen. Durch den einfachen Aufbau und den entsprechenden Materialeinsatz ist die Kryospitze kostengünstig herstellbar. Sowohl die Diamantvariante als auch die beschichteten Kryospitzen bieten den Vorteil, dass ein potenzielles Anhaften des abgekühlten Gewebes an der Kryospitze unterbleibt.

Die erfindungsgemäße Kryospitze kann mit sehr kleinen Abmessungen hergestellt werden, sodass bei ihrer Anwendung umliegendes und gesundes Gewebe geschont wird. Die Kryospitze kann aus jedem geeigneten Material hergestellt werden. Üblicherweise werden derartige Vorrichtungen aus Glas oder Glaskeramik hergestellt. Für die erfindungsgemäße Kryospitze sind auch Kunststoffe, z. B. faserverstärkte Kunststoffe geeignet, welche exzellente Wärmeisolationseigenschaften aufweisen. Die notwendigen feinen Strukturen der erfindungsgemäßen Kryospitze können mit Verfahren, die zur Herstellung sehr feiner Strukturen geeignet sind, hergestellt werden, z. B. durch Feinstfräsen. Auch Spritzgussverfahren, 3D-Druck und andere Prototypingverfahren kommen inbetracht. Dazu können z. B. zwei Hälften mit den entsprechenden Bohrungen hergestellt und anschließend formschlüssig zusammengefügt werden.

Insgesamt wird durch die erfindungsgemäße Lösung das Risiko der mechanischen und thermischen Beanspruchung des umliegenden gesunden Gewebes minimiert.

Überraschenderweise wurde festgestellt, dass die Handhabbarkeit der Kryospitze dadurch erleichtert wird, dass die Expansion des Kryofluids bei der direkten Vereisungsvariante lediglich innerhalb eines kraftschlüssigen Volumens stattfindet und nicht außerhalb der Einrichtung im Gewebe, wie es im Stand der Technik beschrieben wurde. Dort findet die Gasexpansion innerhalb des Gewebes unter Druck in alle Richtungen statt und erschwert somit die Handhabung der Kryoeinrichtung. Durch die erfindungsgemäße Kryospitze existiert die geschlossene Kryofluidexpansion und durch die besondere Ausführungsform der Fluidrückführung nur ein einziger minimaler mechanischer Widerstand entgegen der Normalkraft für den Operateur. Somit ist die direkte selektive Aufbringung der Kältestellen besser möglich als bisher.

Die Zeichnungen zeigen Ausführungsbeispiele für die erfindungsgemäße Kryospitze: Es zeigen
Fig. 1: eine erfindungsgemäße Kryospitze.
Fig. 2a: eine erfindungsgemäße Kryospitze mit Ableitungskanal.
Fig 2b: eine erfindungsgemäße Kryospitze mit Ableitungskanal zur indirekten Kryotherapie.
Fig. 3: eine erfindungsgemäße Kryospitze zur Behandlung der Sklera.
Fig. 4: Teilansicht einer erfindungsgemäßen Kryospitze.
Fig. 5: eine erfindungsgemäße Kryospitze mit Dosiereinrichtung.

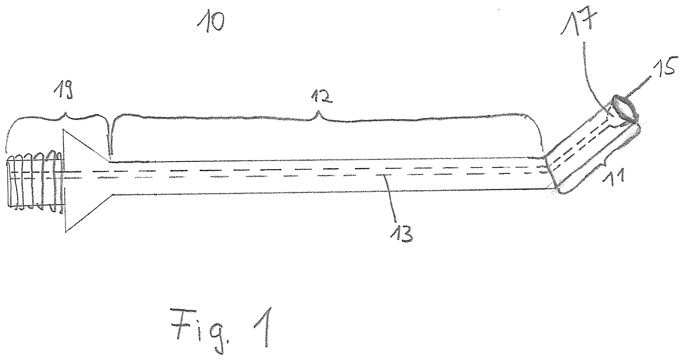

Im Folgenden wird bezugnehmend auf Fig. 1 eine Ausführungsform der Kryospitze 10 beschrieben. Die Ausführungen gelten auch, soweit sinnvoll, für andere Ausgestaltungen der Kryospitze.

Die Kryospitze 10 weist einen röhrenförmigen Körper auf. Die Gestaltung des Querschnitts des röhrenförmigen Körpers ist unkritisch und er kann über die gesamte Länge gleich oder variabel sein. In der Regel wird der röhrenförmige Körper so gestaltet, dass er einfach herzustellen ist und die Funktionen ausüben kann, die notwendig sind. Der Querschnitt kann eine beliebige Form haben, z. B. eckig, ellipsenförmig, polygon. In einer bevorzugten Ausführungsform der Kryospitze 10 ist der Querschnitt des röhrenförmigen Körpers ellipsenförmig, insbesondere im Wesentlichen rund.

Die Kryospitze 10 weist einen röhrenförmigen Körper mit einem Mittelteil, einem distalen Ende 11 und einem proximalen Ende 19 auf. Am distalen Ende des röhrenförmigen Körpers befindet sich eine Verbreiterung 17. Die Kryospitze weist weiterhin einen Fluidkanal 13 innerhalb des röhrenförmigen Körpers auf, der innerhalb des röhrenförmigen Körpers vom proximalen Ende 19 über den Mittelteil 12 bis hin zum distalen Ende 11 verläuft und geeignet ist um ein unter Druck stehendes kryogenes Fluid zu leiten. Am offenen Abschluss des distalen Endes 11 befindet sich ein elastischer Rand, welcher kraftschlüssig auf das zu behandelnde Gewebe aufgebracht werden kann. Weiterhin befindet sich innerhalb des distalen Endes eine Verbreiterung 17 des Fluidkanal, durch die das Fluidvolumen expandieren kann und um die Grenzfläche zwischen kryogenem Fluid und dem zu behandelnden Gewebe zu erhöhen. Der elastische Rand 15 dient dazu eine besonders gute Abdichtung zu erreichen.

Die relative Länge der Verbreiterung 17 am distalen Ende 11 zur Gesamtlänge der Kryospitze beträgt zwischen 1/16 - 1/4. In einer bevorzugten Ausführungsform beträgt die relative Länge des distalen Endes 11 zur Gesamtlänge der Ausführungsform der Kryospitze 10 zwischen 1/12 - 1/5. In einer besonders bevorzugten Ausführungsform beträgt die relative Länge des distalen Endes 11 zur Gesamtlänge der Ausführungsform der Kryospitze 10 der zwischen 1/10 - 1/8.

Die Form der Verbreiterung 17, welche zur Vergrößerung der Grenzfläche zwischen dem kryogenen Fluid und dem zu behandelnden Gewebe, der Fluidzuführung und der Fluidableitung dient, ist unkritisch. Geeignet sind beispielsweise ein Teilellipsoid oder eine andere ununterbrochene geometrische Form. In einer bevorzugten Ausführungsform ist die Verbreiterung 17 ein Teilellipsoid.

Das proximale Ende 19 ist so ausgestaltet, dass eine Zuleitung für das Fluid und ggf. eine Dosiereinheit angeschlossen werden kann. Die relative Länge des proximalen Endes 19 zur Gesamtlänge der Kryospitze beträgt zwischen 1/16 und 1/2. In einer bevorzugten Ausführungsform beträgt die relative Länge des proximalen Endes zur Gesamtlänge der Kryospitze zwischen 1/8 - 1/3. In einer besonders bevorzugten Ausführungsform beträgt die relative Länge des proximalen Endes 19 zur Gesamtlänge der Kryospitze 10 zwischen 1/4 - 1/3.

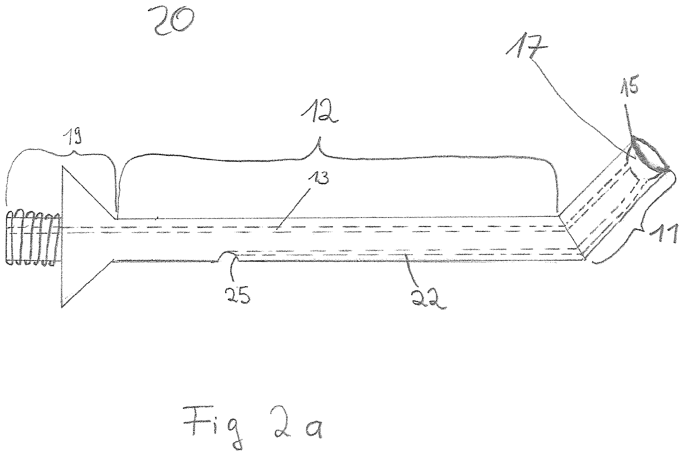

In Figur 2a wird eine weitere Ausführungsform der Kryospitze 20 gezeigt. Hierbei ist ein Ableitungskanal 22 mit der Verbreiterung 17 verbindbar, welcher ein Druckgefälle gegenüber Fluidkanal 13 aufweist, damit das expandierte kryogene Fluid aus der kraftschlüssigen Verbindung zwischen dem elastischen Rand 15 und dem Gewebe abgeleitet werden kann.

Das expandierte Fluid kann über eine Auslassöffnung 25, die sich näher dem proximalen Teil der Kryospitze 20 befindet, an die Umgebung abgegeben werden. Die Auslassöffnung 25 befindet sich außerhalb des zu behandelnden Gewebes. In einer bevorzugten Ausführungsform befindet sich die Auslassöffnung 25 zudem ausreichend entfernt von dem umliegenden gesunden Gewebe, um dieses durch das austretende Fluid weder thermisch noch mechanisch zu belasten. In einer besonders bevorzugten Ausführungsform befindet sich die Auslassöffnung 25 in dem Drittel des Mittelteils 12, welches sich näher am proximalen Ende 19 befindet.

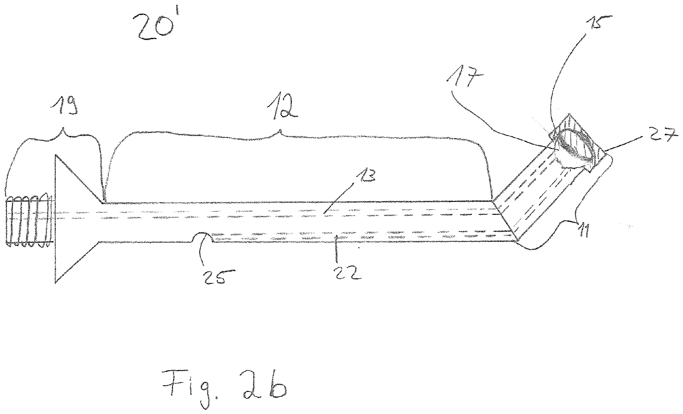

Somit können bei der kryotherapeutischen Behandlung kein Stofftransport und kein Wärmetransport außerhalb des Kraftschlusses mit dem umliegenden gesunden Gewebe stattfinden. Lediglich an der Grenzfläche, welche durch den Kraftschluss zustande kommt, zwischen Fluid und dem zu behandelnden Gewebe findet die Kühlung statt. Durch die besondere Ausführungsform 20 in Figur 2 kann das Fluid bei der kryotherapeutischen Behandlung des zu behandelnden Gewebes innerhalb des Kraftschlusses entspannen. Der Widerstand durch die Gasexpansion für den Operateur bei der Handhabung der Kryospitze während der Expansion des Fluids wird durch den Druckausgleich über den Ableitungskanal 22 und die Auslassöffnung 25 minimal.

Die thermische Belastung des gesunden Gewebes, bei der Rückführung des gebrauchten Kryofluids innerhalb der Kryospitze 20 nach der Behandlung von tiefliegendem zu behandelndem Gewebe, wird ferner dadurch vermieden, dass die Kryospitze 20 aus einem wärmeisolierenden Material besteht. In einer bevorzugten Ausführungsform wird zur Herstellung der Kryospitze 20 ein Polymer verwendet. Jedes die Bedingungen in der Spitze tolerierende, und gegenüber dem Gewebe inerte Polymer ist geeignet. Nützlich sind außerdem solche Polymere, die sich mechanisch so bearbeiten lassen, dass sehr feine Strukturen entstehen können. Geignet sind insbesondere kältetolerante Polymere wie Polyetheretherketon (PEEK) und flurorierte und teilfluorierte Polymere. Beispiele sind PTFE, Perfluoralkoxy-Polymere (PFA), Ethylen-Tetrafluorethylen-Copolymer (ETFE) und weitere teilfluorierte und vollständig fluorierte Polymere bzw. Polycarbonat, Polyetheretherketon (PEEK) oder mit Glas verstärktem Polyetheretherketon. Die Kryospitze kann ganz aus einem Polymer bestehen, es können auch Mischungen von Polymeren verwendet werden. Ebenso ist es möglich, dass die einzelnen Teile der Spitze aus verschiedenen Polymeren, jeweils angepasst an die Bedürfnisse, hergestellt werden. Bevorzugt wird für die Kryospitze ein Material verwendet.

In einer Ausführungsform kann die Herstellung der Kryospitze aus einem wärmeisolierenden Materialien über die Erzeugung zweier formschlüssig verbindbarer Hälften eines wärmeisolierenden Materials erfolgen, welche mit Feinstfräsen oder über ein Spritzgussverfahren hergestellt werden können. In einer anderen Ausführungsform können die formschlüssig verbindbaren Hälften eines wärmeisolierenden Materials mit Feinstfräsen hergestellt werden. Es ist auch möglich, die formschlüssig verbindbaren Hälften eines entsprechenden wärmeisolierenden Materials über ein Spritzgussverfahren herzustellen. Die beiden ausgearbeiteten formschlüssig verbindbaren Hälften eines entsprechenden wärmeisolierenden Materials können dann in einem weiteren Herstellungsschritt formschlüssig miteinander verbunden werden.

Die relative Länge des distalen Endes 11 zur Gesamtlänge der Ausführungsform der Kryospitze 20 beträgt zwischen 1/16 - 1/4. In einer bevorzugten Ausführungsform beträgt die relative Länge des distalen Endes 11 zur Gesamtlänge der Ausführungsform der Kryospitze 20 zwischen 1/12 - 1/5. In einer besonders bevorzugten Ausführungsform beträgt die relative Länge des distalen Endes 11 zur Gesamtlänge der Ausführungsform der Kryospitze 20 zwischen 1/10 - 1/8.

Die relative Länge des proximalen Endes 19 zur Gesamtlänge der Ausführungsform der Kryospitze 20 beträgt zwischen 1/16 und 1/2. In einer bevorzugten Ausführungsform beträgt die relative Länge des proximalen Endes zur Gesamtlänge der Ausführungsform der Kryospitze 20 zwischen 1/8 - 1/3. In einer besonders bevorzugten Ausführungsform beträgt die relative Länge des proximalen Endes 19 zur Gesamtlänge der Ausführungsform der Kryospitze 20 zwischen 1/4 - 1/3.

Eine weitere Ausführungsform der Kryospitze 20' wird in Figur 2b gezeigt. Die Kryospitze 20' gleicht der Kryospitze 20 aus Figur 2a, wobei die Kryospitze 20' ergänzend eine Verschlusskappe 27 am distalen Ende 11 aufweist. Die Verbindung zwischen distalem Ende 11 und der Verschlusskappe ist gasdicht und formschlüssig. Die Verschlusskappe 27 ermöglicht eine indirekte Vereisung des zu behandelnden Gewebes. Diese Ausführungsform ist besonders für zu behandelndes Gewebe geeignet, das schonend, d. h. mit einer geringeren Abkühlungsgeschwindigkeit gekühlt werden muss, um beispielsweise thermische Spannungen mit dem umliegenden gesunden Gewebe zu vermeiden.

Durch die Verschlusskappe 27 kommt ein Wärmedurchgang zustande, wodurch das kryogene Fluid nicht mehr in direktem Kontakt zum zu behandelnden Gewebe steht, wobei die Abkühlgeschwindigkeit proportional zur Vergrößerung der Wandstärke und der Beschaffenheit der Verschlusskappe 27 abnimmt. Die Verschlusskappe 27 ist aus Material mit guten Wärmeleitfähigkeitseigenschaften gebildet. Dabei ist es wichtig, dass das wärmeleitfähige Material, zumindest soweit es mit dem Fluid und dem Körper in Berührung kommt, biokompatibel ist. Die Verschlusskappe kann ein oder mehrere Schichten aufweisen, wobei die Schichten unterschiedlich in ihrer Dimension und/oder in ihren Eigenschaften sein können. In einer Ausführungsform besteht die Verschlusskappe aus einem Grundkörper, der auf einer Seite oder auf beiden Seiten oder insgesamt mit einer oder mehreren Schichten überzogen ist. Der Grundkörper kann aus Metall, einer Metalllegierung, aus Diamant oder Polymer gebildet sein. In einer Ausführungsform besteht die Verschlusskappe 27 aus Diamant oder Metall. In einer weiteren Ausführungsform besteht der Grundkörper aus einem wärmeleitfähigen Material, wie Metall oder einem Polymer und weist zusätzlich eine Polymerschicht auf, die den Grundkörper, der z. B. aus Metall oder einer Metalllegierung besteht, zumindest teilweise überzieht. Der Überzug kann Eigenschaften wie bessere Verträglichkeit beitragen. In einer bevorzugten Ausführungsform ist die Verschlusskappe 27 aus einer Metalllegierung, z. B. Edelstahl, oder aus Diamant.

Die Verschlusskappe 27 kann am distalen Ende auf die Verbreiterung des Fluidkanals aufgesetzt werden. Die Kappe wird so aufgebracht, dass sie während der Behandlung an Ort und Stelle bleibt und sich nicht verschiebt oder entfernt wird. Dazu kann die Kappe in an sich bekannter Weise fixiert werden, z. B. aufgesteckt, geschraubt, geklebt oder in einer sonstigen festen Verbindung angebracht werden.

Die Verschlusskappe kann entsprechend der vorgesehenen Art der Behandlung gestaltet werden. Durch ihre Größe und die Wandstärke kann die Abkühlgeschwindigkeit des Gewebes variiert werden. Zudem kann die Geometrie der Spitze so ausgeformt sein, dass eine bestimmte Fläche gekühlt wird (zylindrisch, ellipsoid, spitz).

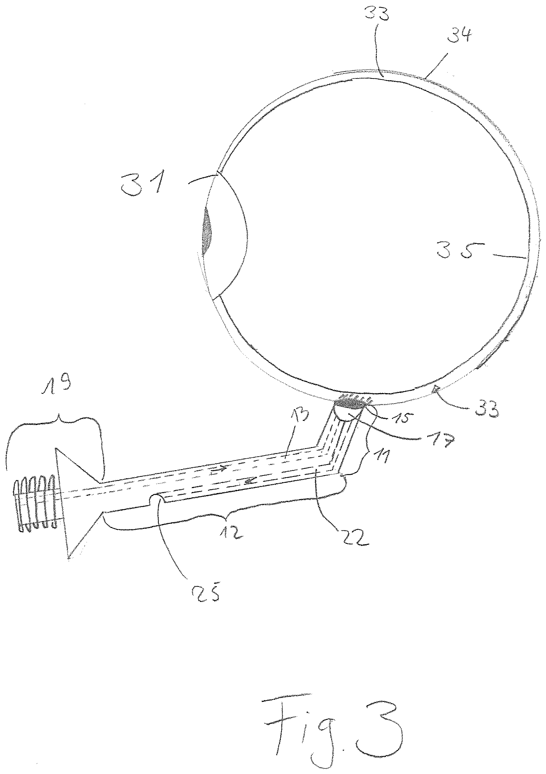

Figur 3 zeigt eine bevorzugte Ausführungsform der Kryospitze 30, bei der das zu behandelnde Gewebe die Sklera 33 des Auges 31 ist. In einer bevorzugten Ausführungsform können Kälteherde an dem zu behandelnden Gewebe der äußeren Stellen 34 der Sklera 33, welche ohne Inzision zugänglich sind, oder an dem zu behandelnden Gewebe der tieferliegenden Gewebeschichten 35 der Sklera 33, welche durch eine Inzision zugänglich sind, gesetzt werden.

Die Dimensionen der Kryospitze werden so gewählt, dass sie für den vorgesehenen Zweck geeignet sind. So weist zum Beispiel für einen Einsatz am Auge die Kryospitze einen möglichst geringen Querschnitt auf, um möglichst wenig Gewebe zu vereisen. Geeignet ist beispielsweise, dass die Außendurchmesser des distalen Endes 11 und des Mittelteils 12 identisch sind und zwischen 1,5 mm und 5 mm liegen. Falls eine Verschlusskappe 27 vorgesehen ist, können die Außendurchmesser weiter reduziert werden und im Bereich von unter 1 mm liegen.

In einer bevorzugten Ausführungsform weisen die Außendurchmesser des distalen Endes 11 und des Mittelteils 12 zwischen 2,0 und 4 mm auf. In einer besonders bevorzugten Ausführungsform weisen die Außendurchmesser des distalen Endes 11 und des Mittelteils zwischen 2,5 und 3,5 mm auf.

Die Gesamtlänge der Kryospitze hängt von dem Behandlungsort ab. Je tiefer der Einsatzort im Gewebe ist, je länger muss die Spitze sein. Der Fachmann kann die jeweils am besten geeigneten Dimensionen leicht feststellen. Geeignet für die Verwendung am Auge ist z. B. eine Länge von 20 bis 90 mm. In einer bevorzugten Ausführungsform beträgt daher die Gesamtlänge der Kryospitze zwischen 30 und 80 mm. In einer besonders bevorzugten Ausführungsform beträgt die Gesamtlänge der Kryospitze zwischen 40 und 70 mm.

Das kryogene Fluid dient dazu das zu behandelnde Gewebe zu vereisen. Als kryogene Fluide für alle Ausführungsformen kommen alle dem Fachmann zur kryotherapeutischen Behandlung bekannten kryogenen Fluide infrage. Das Kryogene Fluid ist entweder eine Flüssigkeit oder ein Gas (z. B. N₂, N₂O, CO₂ Halogenkohlenwasserstoffe mit einem Siedebereich zwischen -30 C und -41 C)). In einer bevorzugten Ausführungsform ist das kryogene Fluid ein Gas (z. B. N₂, N₂O, CO₂ oder Halogenkohlenwasserstoffe mit einem Siedebereich zwischen -30 C und -41 C)). In einer besonders bevorzugten Ausführungsform ist das Gas N₂O.

Durch die Expansion eines unter Druck stehenden Gases an einer Drossel (beispielsweise ein Ventil), in ein Volumen, das unter einem niedrigeren Druck steht, findet eine Gaskühlung aufgrund des Joule-Thomson-Effekts statt (adiabatische, isenthalpische Expansion eines realen Gases), welcher dem Fachmann hinreichend bekannt ist. Hierbei ist die Einrichtung zur Gasexpansion hinreichend thermisch isoliert. Die vorliegende Erfindung kühlt das zu behandelnde Gewebe über die adiabatische, isenthalpische Expansion eines Gases in der Kryospitze, welche thermisch hinreichend isoliert ist. Das verwendete kryogene Gas wird auf die jeweilige Behandlungstemperatur bzw. die erforderliche Kühlgeschwindigkeit, welche für das zu behandelnde Gewebe geeignet ist, angepasst. Mit N₂ können Gastemperaturen von bis zu -196C, mit N₂O können Gastemperaturen von bis zu -89 C, mit CO₂ können Gastemperaturen von bis zu -79C und mit entsprechenden Halogenkohlenwasserstoffen Gastemperaturen von bis zu -41 C erreicht werden.

Die erfindungsgemäße Kryospitze kann, wie in Figur 3 gezeigt, einen modularen Aufbau aufweisen, wobei die Spitze mit und ohne eine Verschlusskappe 27, betrieben werden kann. Die Verschlusskappe wird zur Dosierung der Kälte und wenn eine schonendere Abkühlung notwendig ist, empfohlen. Hierbei ist die Verschlusskappe 27 analog zu Fig. 2b gasdicht und formschlüssig mit dem distalen Ende 11 verbindbar.

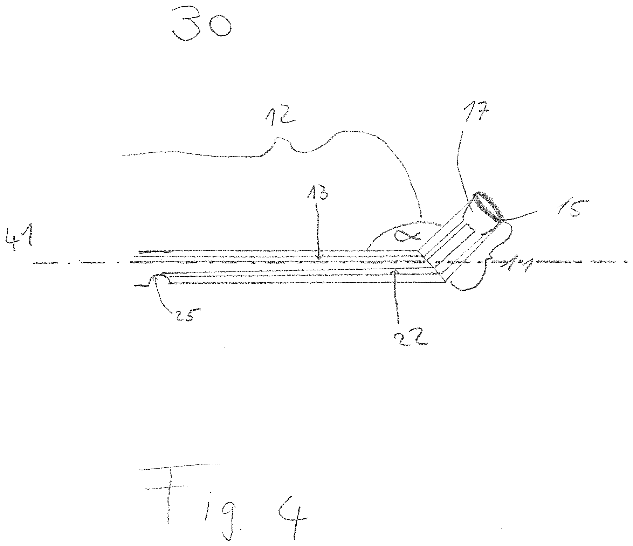

Figur 4 zeigt eine Teilansicht der Ausführungsform der Kryospitze 30, in der das distale Ende 11 und das Mittelteil 12 in einem Winkel zwischen 90° und 180° miteinander verbunden sind. Das distale Ende 11 ist hierbei in einem Winkel gegenüber der Längsachse 41 mit Mittelteil 12 verbunden. In einer bevorzugten Ausführungsform ist das distale Ende 11 in einem stumpfen Winkel α mit Mittelteil 12 verbunden. In einer besonders bevorzugten Ausführungsform beträgt der Winkel α zwischen 150 und 120 .

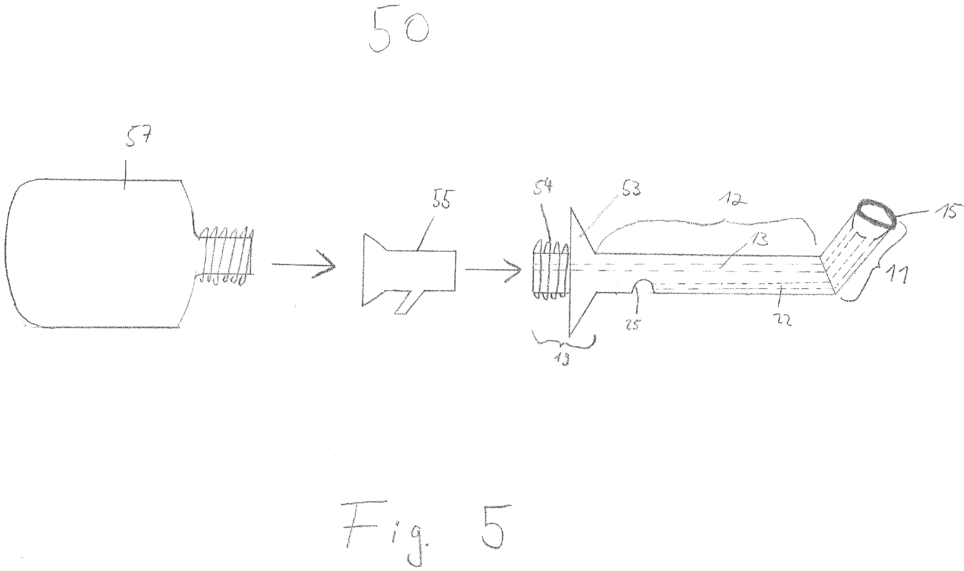

Die erfindungsgemäße Kryospitze kann, wie in Figur 5 gezeigt, am proximalen Ende 19 ein Verbindungsglied mit einem Außengewinde 54 aufweisen, um weitere Einrichtungen anzuschließen. In einer bevorzugten Ausführungsform kann an das Verbindungsglied 55 eine Dosiereinrichtung, die zum Beispiel ein manuelles Ventil sein kann, angeschlossen werden. Über Verbindungsglied 55 an dem die adiabatische, isenthalpische Gasexpansion stattfindet, kann das unter Druck stehende kryogene Fluid kontrolliert der Kryospitze zugeführt werden.

Das Verbindungsglied 55 mit der Ausführungsform 30 der Kryospitze ist in einer weiteren Ausführungsform mit einem Gasbehälter 57 verbindbar. Der Gasbehälter 57 ist ein unter Druck stehendes Behältnis, das als Vorratsbehältnis für das Kältefluid dient. Die Ausgestaltung der erfindungsgemäßen Kryospitze ermöglicht es, dass jeder bekannte Gasbehälter angeschlossen werden kann. Insbesondere ist es möglich als Gasbehälter 57 eine kleine Gaskartusche, eine Gaspatrone, eine Gaskapsel oder eine kleine Gasflasche zu verwenden. In dieser Ausführungsform ist die Kryospitze besonders gut geeignet für den Einsatz im Rahmen einer Operation.

Die Kryospitze 30 aus Figur 5 kann durch ihren modularen Aufbau in einer weiteren Ausführungsform eine Verschlusskappe 27 aufweisen, wenn eine schonendere Abkühlung durch die indirekte Variante der Kryoapplikation notwendig ist.

Durch die Verbindung mit einem kleinen Gasbehälter 57 und einem Verbindungsglied 55 kann die Kryospitze leicht handhabbar und mit geringem Gewicht direkt und bequem an das zu behandelnde Gewebe herangeführt werden. Hierbei fallen die Nachteile konventioneller kryotherapeutischer Geräte, welche einen komplexen Aufbau mit einer unhandlicheren Peripherie aufweisen, weg.

In einer bevorzugten Ausführungsform weist das proximale Ende 19 eine geometrische Form 53 vor dem Außengewinde 54 auf. Diese Einrichtung dient zur Druckkompensation am proximalen Ende 54, wo die Fluidentspannung in den Fluidkanal 13 nach der Drosselung stattfindet. In einer besonders bevorzugten Ausführungsform ist die geometrische Form 53 ein Kegelstumpf.

## Patentansprüche

1. Kryospitze mit distalem und proximalem Ende zur therapeutischen Kältebehandlung von Gewebe, die aufweist:
einen röhrenförmigen Körper und einen Fluidkanal, der innerhalb der Kryospitze das proximale und das distale Ende verbindet und so ausgebildet ist, dass er ein kryogenes Fluid leiten kann;
eine Verbreiterung des Fluidkanals am distalen Ende, welche am Abschluss des distalen Endes der Kryospitze, der einen elastischen Rand aufweist, in eine Öffnung mündet, **dadurch gekennzeichnet**, das besagte Öffnung derart gestaltet ist, dass sie kraftschlüssig auf das zu behandelnde Gewebe aufgebracht werden kann.

2. Kryospitze nach Anspruch 1, **dadurch gekennzeichnet, dass** sie einen innenliegenden Ableitungskanal aufweist, welcher mit der Verbreiterung verbindbar ist und die Rückführung des gebrauchten kryogenen Fluids in Richtung des proximalen Endes ermöglicht.

3. Kryospitze nach einem der Ansprüche 1 oder 2, **dadurch gekennzeichnet, dass** die Kryospitze eine Auslassöffnung aufweist, wobei sich die Auslassöffnung oberhalb des zu behandelnden Gewebes befindet.

4. Kryospitze nach einem der vorhergehenden Ansprüchen, **dadurch gekennzeichnet, dass** das Gewebe die Sklera des Auges ist.

5. Kryospitze nach einem der vorhergehenden Ansprüche, **dadurch gekennzeichnet, dass** das kryogene Fluid ein Gas ist.

6. Kryospitze nach einem der vorhergehenden Ansprüchen, **dadurch gekennzeichnet, dass** die Kryospitze einen Außendurchmesser zwischen 1,5 mm und 5 mm aufweist.

7. Kryospitze nach einem der vorhergehenden Ansprüche, **dadurch gekennzeichnet, dass** das distale Ende gegenüber der Längsachse gewinkelt ist, wobei der Winkel bevorzugt ein stumpfer Winkel ist.

8. Kryospitze nach Ansprüchen 1-4, **dadurch gekennzeichnet, dass** die Verbreiterung ein Teilellipsoid oder eine andere ununterbrochene geometrische Form ist.

9. Kryospitze nach Ansprüchen 1-8, **dadurch gekennzeichnet, dass** die Kryospitze am proximalen Ende ein Verbindungsglied aufweist, wobei optional das Verbindungsglied mit einem Gasbehälter verbindbar ist.

10. Kryospitze nach Anspruch 9, **dadurch gekennzeichnet, dass** das Verbindungsglied eine Dosiereinrichtung ist.

11. Kryospitze nach Ansprüchen 1-10, **dadurch gekennzeichnet, dass** das proximale Ende ein Außengewinde aufweist.

12. Kryospitze nach einem der Ansprüche 1-11, **dadurch gekennzeichnet, dass** die Kryospitze eine geometrische Erweiterung vor dem Außengewinde aufweist.

13. Kryospitze nach einem der Ansprüche 1-12, wobei die Kryospitze zumindest teilweise aus einem thermisch isolierenden Material besteht, wobei das thermisch isolierende Material ein Kunststoff oder ein faserverstärkter Kunststoff, PTFE, ein Perfluoralkoxy-Polymere (PFA), ein Ethylen-Tetrafluorethylen-Copolymer (ETFE), ein teilfluoriertes oder vollständig fluoriertes Polymer, oder Polycarbonat, Polyetheretherketon (PEEK) oder mit Glas verstärktes Polyetheretherketon sein kann.

14. Kryospitze nach einem der Ansprüche 1-13, wobei die Kryospitze eine zusätzliche Verschlusskappe aufweist, welche mit dem distalen Ende verbindbar ist, wobei bevorzugt die Verschlusskappe zumindest teilweise aus einem Material mit guten Wärmeleitfähigkeitseigenschaften besteht oder damit überzogen ist und wobei die Verschlusskappe aus Metall, einer Metalllegierung oder Diamant bestehen kann.

15. Verfahren zur Herstellung einer Kryospitze nach Anspruch 1, umfassend:
Erzeugung zweier formschlüssig verbindbarer Hälften, zumindest teilweise bestehend aus einem wärmeisolierenden Material mit je einer länglichen Aussparung,
Zusammenfügen der beiden formschlüssig verbindbaren Hälften der Kryospitze unter Bildung eines Fluidkanals.

## Claims

1. Cryo tip with a distal and a proximal end for therapeutic cold treatment of tissue, comprising:
a tubular body and a fluid channel connecting the proximal and the distal end within the cryo tip and adapted to guide a cryogenic fluid;
a widening of the fluid channel at the distal end, which leads into an opening at the end of the distal end of the cryo tip having an elastic edge, **characterized in that** said opening is designed such that it can be applied frictionally-locking on the tissue to be treated.

2. Cryo tip according to claim 1, **characterized in that** it comprises an internal discharge channel, which is connectable to the widening and allows the return of the used cryogenic fluid in the direction of the proximal end.

3. Cryo tip according to one of claims 1 or 2, **characterized in that** the cryo tip comprises an outlet opening, wherein the outlet opening is located above the tissue to be treated.

4. Cryo tip according to one of the preceding claims, **characterized in that** the tissue is the sclera of the eye.

5. Cryo tip according to one of the preceding claims, **characterized in that** the cryogenic fluid is a gas.

6. Cryo tip according to one of the preceding claims, **characterized in that** the cryo tip has an outer diameter between 1.5 mm and 5 mm.

7. Cryo tip according to one of the preceding claims, **characterized in that** the distal end is angled relative to the longitudinal axis, wherein the angle is preferably an obtuse angle.

8. Cryo tip according to claims 1-4, **characterized in that** the widening is a partial ellipsoid or another uninterrupted geometric shape.

9. Cryo tip according to claims 1-8, **characterized in that** the cryo tip comprises a connecting member at the proximal end, wherein optionally the connecting member is connectable to a gas container.

10. Cryo tip according to claim 9, **characterized in that** the connecting member is a metering device.

11. Cryo tip according to claims 1-10, **characterized in that** the proximal end has an external thread.

12. Cryo tip according to one of claims 1-11, **characterized in that** the cryo tip comprises a geometric extension in front of the external thread.

13. Cryo tip according to one of claims 1-12, wherein the cryo tip is at least partially made of a thermally insulating material, wherein the thermally insulating material can be a plastic or a fiber-reinforced plastic, PTFE, a perfluoroalkoxy polymer (PFA), an ethylene tetrafluoroethylene (ETFE), a partially fluorinated or fully fluorinated polymer, or polycarbonate, polyether ether ketone (PEEK) or glass reinforced polyether ether ketone.

14. Cryo tip according to one of claims 1-13, wherein the cryo tip comprises an additional cap which is connectable to the distal end, wherein preferably the cap is at least partially made of a material having good thermal conductivity properties or coated therewith and wherein the cap can be made of metal, a metal alloy or diamond.

15. Method for producing a cryo tip according to claim 1, comprising:
generating two form-locking connectable halves, at least partially consisting of a heat-insulating material, each with an elongated recess,
assembling the two form-locking connectable halves of the cryo tip to form a fluid channel.

## Revendications

1. Pointe cryogénique avec une extrémité distale et une extrémité proximale pour le traitement thérapeutique par le froid de tissus, qui présente:
un corps tubulaire et un canal de fluide, qui relie à l'intérieur de la pointe cryogénique l'extrémité proximale et l'extrémité distale et qui est configuré de façon à pouvoir transporter un fluide cryogénique;
un élargissement du canal de fluide à l'extrémité distale, qui débouche dans une ouverture à la fin de l'extrémité distale de la pointe cryogénique, qui présente un bord élastique,
**caractérisée en ce que** ladite ouverture est configurée de telle manière qu'elle puisse être appliquée par force sur le tissu à traiter.

2. Pointe cryogénique selon la revendication 1, **caractérisée en ce qu'**elle présente un canal d'évacuation interne, qui peut être relié à l'élargissement et qui permet le retour du fluide cryogénique utilisé en direction de l'extrémité proximale.

3. Pointe cryogénique selon une des revendications 1 ou 2, **caractérisée en ce que** la pointe cryogénique présente une ouverture de sortie, l'ouverture de sortie se trouvant au-dessus du tissu à traiter.

4. Pointe cryogénique selon l'une quelconque des revendications précédentes, **caractérisée en ce que** le tissu est la sclérotique de l'oeil.

5. Pointe cryogénique selon l'une quelconque des revendications précédentes, **caractérisée en ce que** le fluide cryogénique est un gaz.

6. Pointe cryogénique selon l'une quelconque des revendications précédentes, **caractérisée en ce que** la pointe cryogénique présente un diamètre extérieur compris entre 1,5 mm et 5 mm.

7. Pointe cryogénique selon l'une quelconque des revendications précédentes, **caractérisée en ce que** l'extrémité distale est coudée par rapport à l'axe longitudinal, dans laquelle l'angle est de préférence un angle obtus.

8. Pointe cryogénique selon les revendications 1 à 4, **caractérisée en ce que** l'élargissement est un ellipsoïde partiel ou une autre forme géométrique ininterrompue.

9. Pointe cryogénique selon les revendications 1 à 8, **caractérisée en ce que** la pointe cryogénique présente à l'extrémité proximale un organe de raccordement, l'organe de raccordement pouvant en option être raccordé à un réservoir de gaz.

10. Pointe cryogénique selon la revendication 9, **caractérisée en ce que** l'organe de raccordement est un dispositif de dosage.

11. Pointe cryogénique selon les revendications 1 à 10, **caractérisée en ce que** l'extrémité proximale présente un filet extérieur.

12. Pointe cryogénique selon l'une quelconque des revendications 1 à 11, **caractérisée en ce que** la pointe cryogénique présente un élargissement géométrique avant le filet extérieur.

13. Pointe cryogénique selon l'une quelconque des revendications 1 à 12, la pointe cryogénique se composant au moins en partie d'un matériau thermo-isolant, dans laquelle le matériau thermo-isolant peut être une matière plastique ou une matière plastique renforcée par des fibres, PTFE, un perfluoroalcoxy-polymère (PFA), un copolymère éthylène-tétra-fluoroéthylène (ETFE), un polymère partiellement fluoré ou complètement fluoré, ou un polycarbonate, une polyétheréthercétone (PEEK), ou une polyétheréthercétone renforcée par du verre.

14. Pointe cryogénique selon l'une quelconque des revendications 1 à 13, la pointe cryogénique présentant un capuchon de fermeture supplémentaire, qui peut être assemblé avec l'extrémité distale, dans laquelle le capuchon de fermeture est de préférence constitué au moins en partie d'un matériau possédant de bonnes propriétés de conductibilité thermique ou en est revêtu, et dans laquelle le capuchon de fermeture peut être constitué de métal, d'un alliage métallique ou de diamant.

15. Procédé de fabrication d'une pointe cryogénique selon la revendication 1, comprenant:
la production de deux moitiés pouvant être assemblées par emboîtement, se composant au moins en partie d'un matériau thermo-isolant, dotées chacune d'une découpe allongée,
la jonction des deux moitiés de la pointe cryogénique pouvant être assemblées par emboîtement, avec formation d'un canal de fluide.
